# EUROPEAN PATENT APPLICATION

(11) **EP 3 643 238 A1**
(43) Date of publication of application: **29.04.2020**
(21) Application number: 18290124.9
(22) Date of filing: 25.10.2018
(51) Int. Cl.: A61B 6/00, A61B 6/12, G06T 7/00

(54) **IMAGE BASED GUIDING OF AN INTERVENTIONAL DEVICE**

(71) Applicant: Koninklijke Philips N.V., 5656 AG Eindhoven (NL)
(72) Inventor: Auvray, Vincent Maurice André, 92150 Suresnes Cedex (FR); Florent, Raoul, 92150 Suresnes Cedex (FR)
(74) Representative: de Haan, Poul Erik

(57) **Abstract**

The present invention relates to guiding an interventional device. In order to provide improved guidance when navigating by the aid of roadmapping, an apparatus (10) for guidance of an interventional device is provided. The apparatus comprises an input unit (12); a data storage unit (14), a data processing unit (16) and an output unit (18). The data storage unit is configured to provide a vasculature map of a region of interest with a vasculature presentation of a subject's vasculature structure. The input unit is configured to receive and transfer to the data processing unit at least one current image of the region of interest, wherein an interventional device is at least partly visible in the at least one current image when inserted within the vasculature structure in the region of interest. The data processing unit is configured to combine the vasculature map and the at least one current image, and to detect a location of at least one part of the interventional device in the vasculature map, and to detect if the at least one part of the interventional device lays outside the vasculature presentation shown in the vasculature map and to determine a branching-off location for the interventional device on the vasculature map, and also to adapt the vasculature map in the branching-off location providing an indication of the branching-off location. The output unit is configured to provide an adapted vasculature map.

## Description

### FIELD OF THE INVENTION

The present invention relates to guiding an interventional device, and relates in particular to an apparatus for guidance of an interventional device, to a system for guidance of an interventional device and to a method for guidance of an interventional device in a vasculature structure.

### BACKGROUND OF THE INVENTION

For interventional procedures, devices like catheters or guidewires may be inserted into a subject's body, e.g. into a vascular structure and steered along the extension of the vessels, e.g. to a target region of interest that requires treatment. Such devices may be referred to as interventional devices. Interventional devices may be used, for example, to treat cardiac stenoses. An example of interventional devices are flexible guidewires that are partially or fully radio-opaque. In order to visualize an inserted guidewire during the insertion, i.e. for steering, fluoroscopic low-dose X-ray radiation imaging can be applied. However, in order to also visualize the vessel structure further measures are necessary. For example, contrast agent can be inserted. Another example is to provide diagnostic angiograms that have been acquired with a similar geometrical setup beforehand (i.e. retrospectively acquired), and these images can then be displayed in addition to the live images. Another possibility is to combine the live images with pre-acquired image data of the vasculature by overlaying the live data (such as live image data) to pre-processed angiograms. Such overlay technique between the angiogram and the live images is also known as roadmapping. For example, a roadmap is displayed during navigation to help the clinician to steer his/her interventional tool towards the region of interest. An example for such intervention is percutaneous transluminal coronary angioplasty (PTCA) as applied in catheter laboratories.

For example, US 2013/0322724 A1 describes adaptive roadmapping. However, it has been shown that the device may unwillingly be steered into a vessel not shown on the roadmap. The correction of the movement path by the user for correct steering to a target location may then become cumbersome.

### SUMMARY OF THE INVENTION

There may thus be a need to provide improved guidance of a device when navigating by the aid of roadmapping.

The object of the present invention is solved by the subject-matter of the independent claims; further embodiments are incorporated in the dependent claims. It should be noted that the following described aspects of the invention apply also for the apparatus for guidance of an interventional device, for the system for guidance of an interventional device and for the method for guidance of an interventional device in a vasculature structure.

According to the present invention, an apparatus for guidance of an interventional device is provided. The apparatus comprises an input unit, a data storage unit, a data processing unit and an output unit. The data storage unit is configured to provide a vasculature map of a region of interest with a vasculature presentation of a subject's vasculature structure. The input unit is configured to receive and transfer to the data processing unit at least one current image of the region of interest. An interventional device is at least partly visible in the at least one current image when inserted within the vasculature structure in the region of interest. The data processing unit is configured to combine the vasculature map and the at least one current image. The data processing unit is further configured to detect a location of at least one part of the interventional device in the vasculature map. The data processing unit is also configured to detect if the at least one part of the interventional device lays outside the vasculature presentation shown in the vasculature map and to determine a branching-off location for the interventional device on the vasculature map. The data processing unit is further configured to adapt the vasculature map in the branching-off location providing an indication of the branching-off location. The output unit is configured to provide an adapted vasculature map.

For example, the tip of the interventional device is at least partly visible in the at least one current image and it is detected if the tip lays outside the vasculature presentation shown in the vasculature map.

It is noted that a guide wire fine steering may largely be driven by physical phenomena like blood flow, artery wall mechanical contacts, etc. and that these are also dependent on the guide wire properties. Even if it happens that the guide wire is "attracted" by a secondary vessel, and as the guide wire is steered towards the region of interest, the natural path it follows may drive it into a minor capillary, distracting it from the desired course, the adapted vasculature map provides improved steering information to not only solve the current situation, but also to avoid such situation to happen again.

By determining and indicating the branching-off location, a readable roadmap deprived of too many secondary vessels, is supplemented with additional valuable steering information, since the branching-off location is representing a (smaller) vessel branch.

Thus, roadmapping can be provided that, as a start, only shows the relevant and thus large vessels, where the secondary vessels are removed from the roadmap. However, in view of steering along the vessels, the indication of branching-off location(s) compensates for the missing parts.

Also in cases where due to a lack of sufficient inflow of contrast agent, secondary vessels of interest are invisible in the angiogram, and thus absent from the roadmap, their presence is at least partly indicated by marking the branching-off location(s).

The roadmap is thus adapted only at those locations, where a deviation from the shown vessels occur when steering a device. The adapting is thus kept to a minimum, which also helps in providing a roadmap that shows enough details to provide proper guidance, but which is still readable as a vasculature map. Showing all details for all locations, even if a deviation at those instances does not take place, would lead to a clustered map with reduced guidance properties.

It is noted that the detection of the location of the interventional device, e.g. the tip of the interventional device, is image-based, i.e. based on the 2D current image. Further, also the detection tip of the interventional device lays outside the vasculature shown in the vasculature map and also the determination of the branching-off location for the interventional device on the vasculature map is imaged-based, i.e. based on the 2D vasculature map, or road map. Hence, an image based guidance is provided.

The term vasculature structure refers to a vessel structure. The vasculature map can thus also be referred to as vessel structure map.

The term "branching-off" location refers to a location, where the device is following a vessel structure that is not shown on the presentation of the vasculature map so far. For example, such location is a smaller branch nit shown on the map. The term "branching-off" thus describes the discrepancy of the device actually following the vasculature structure and staying inside the vessels compared to what is shown on the roadmap, i.e. on the vasculature map. With view of the road map, the device is detected as "off road", but the device is actually still "on track" or "on road", just on a smaller road or path, namely the smaller vessel or other vessel not present on the road map so far. The term "branching-off location" can thus also be referred to as "exit location", but it must be noted that this only means the "exit" on the map and not the exit from the vessel itself, as the device maintains inside the vessel. The "branching-off location" can also be referred to as "further location" or next to the vessel-map location".

In an example, the vasculature map is based on at least one angiographic image of the vasculature structure.

According to an example, for providing the indication of the branching-off location, the data processing unit is configured to fine-tune the vasculature map in a predetermined area around the branching-off location based on the at least one angiographic image. For fine-tuning, the data processing unit is configured to detect more detailed vasculature structures in the at least one angiographic image and to add further details of the vasculature structure to the vasculature map to provide an improved vasculature map.

An example for fine-tuning is to add more smaller vessels to the vasculature map.

Another example for fine-tuning is to add more details to vessels already present in the vasculature map.

A further example for fine-tuning is to provide the vessels already present in the vasculature map with a higher resolution.

According to an example, the data processing unit is configured to detect a relevant vascular point or area for the branching-off in the vasculature map. The data processing unit is also configured to transfer the point or area as a relevance location of the branching-off to at least one angiographic image forms a basis for the vasculature map and to extract further details from the at least one angiographic image in the relevance location.

For example, a plurality of angiographic images is used for the roadmap, i.e. the vasculature map. In such case, the relevance location van be transferred to the plurality of angiographic images to extract further details.

The at least one angiographic image has an initial level of details that may not be used for the vasculature map, for example in order to avoid an initial cluttering of the vasculature map by providing too many structures in a too detailed manner.

According to an example, the data processing unit is configured to detect a footprint of the interventional device in the at least one current image. The data processing unit is also configured to transfer the footprint of the interventional device to the vasculature map for the indication of the branching-off location.

In an example, for an adapted indication, the data processing unit is configured to project or present the fine-tuned additions of the vasculature map in the predetermined area(s) only in case of the device tip being present or approaching with a pre-determined distance.

Thus, a clustering of the map is reduced in case of many bifurcations being steered into unintentionally, leading to a larger number of refinement spots on the map.

According to an example, a display unit is provided configured to present the adapted vasculature map.

According to the present invention, also a system for guidance of an interventional device is provided. The system comprises an apparatus for guidance of an interventional device according to one of the preceding examples and a medical imaging device. The medical imaging device provides the current image data of a region of interest of a subject's vasculature structure.

According to an example, the medical imaging device is an X-ray imaging device. The X-ray imaging device is configured to provide X-ray images as the at least one current image of the region of interest. The X-ray imaging device is configured to provide contrast injected X-ray images as the angiographic images of the vasculature structure for the vasculature map.

According to an example, an interventional device is provided adapted to be moved in a steerable manner within a subject's vasculature structure. The interventional device is a steering device for interventional procedures.

According to the present invention, also a method, e.g. a computer-implemented method, for guidance of an interventional device in a vasculature structure is provided. The method comprises the following steps:
a) providing a vasculature map of a region of interest with a vasculature presentation of a subject's vasculature structure;
b) receiving and transferring to a data processing unit at least one current 2D image of the region of interest, wherein an interventional device is at least partly visible in the at least one current image when inserted within the vasculature structure in the region of interest;
c) combining the vasculature map and the at least one current image;
d) detecting a location of at least one part of the interventional device in the vasculature map;
e) detecting if the at least one part of the interventional device lays outside the vasculature presentation shown in the vasculature map and determining a branching-off location for the interventional device on the vasculature map; and
f) adapting the vasculature map in the branching-off location providing an indication of the branching-off location and presenting an adapted vasculature map.

The "providing" of step b) can also be referred to as "receiving and transferring to a processing unit".

According to an aspect, an existing roadmap of a vasculature tree is further refined based on the combination with current images showing a device moved inside the vasculature. In a purely image-based detection and registration procedure, the device is detected in the current images and transferred to the roadmap. It is then assessed, based on the image data, if the device is shown outside the vasculature of the initial roadmap and, hence, if a branching-off from the vasculature as shown in the initial roadmap exists. Under the assumption that the device is always arranged inside a vessel structure, it is assumed that for the present location of the device, a vessel exists. This information is used to update and to adjust the initial roadmap by adding the detected branching-off location as information for the user.

Briefly said, in an example, during navigation of an interventional device, the steering of that device, i.e. the steering device, it is detected whether the device is steered into a vessel absent from the roadmap. A clinician is informed accordingly, e.g. by a displayed image. Further, the displayed map can be updated to represent the missing vessel by refining the roadmap extraction locally, based on the detection of the steering device reaching a vessel not present so far.

Briefly said, secondary vessels are added to a roadmap after a steering device has navigated into them.

In an example, a position of an interventional device that is not equipped with active localization sensors is tracked via image analysis, i.e. via image-based detection. The interventional device is tracked in the image, but without having to construct the track of the device, i.e. without such recursive step.

According to an aspect, an improved roadmapping is provided in which increased vessel information is displayed, i.e. updated information, to support the clinician such that e.g. he/she ensures that the steering device does not enter unintentionally or erroneously in one or more vessels not originally displayed.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of an apparatus for guidance of an interventional device.
Fig. 2 schematically shows an example of a system for guidance of an interventional device.
Fig. 3 schematically shows steps of an example of a method for guidance of an interventional device in a vasculature structure.
Fig. 4 shows a further example of a method for guidance of an interventional device in a vasculature structure.
Fig. 5a shows a guide wire tip branching-offing a roadmap at a non-visible branching-off location; and Fig. 5b shows an example of an adapted vasculature map as an updated roadmap in which the branching-off location is indicated, for example by locally refining the roadmap.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

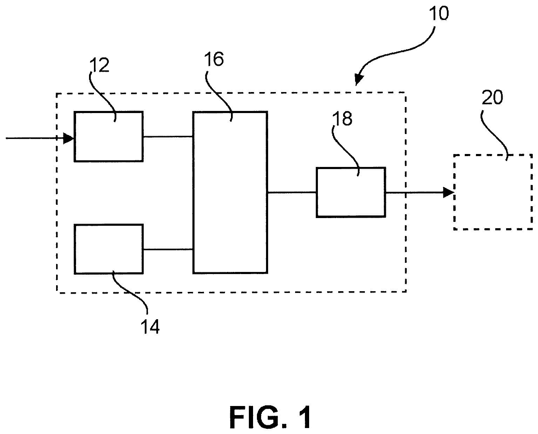

Fig. 1 schematically shows an example of an apparatus 10 for guidance of an interventional device. The apparatus 10 comprises an input unit 12, a data storage unit 14, a data processing unit 16 and an output unit 18. The data storage unit 12 is configured to provide a vasculature map of a region of interest with a vasculature presentation of a subject's vasculature structure. The input unit 14 is configured to receive and transfer to the data processing unit at least one current image of the region of interest. An interventional device is at least partly visible in the at least one current image when inserted within the vasculature structure in the region of interest. The data processing unit 16 is configured to combine the vasculature map and the at least one current image. The data processing unit 16 is also configured to detect a location of at least one part of the interventional device in the vasculature map. The data processing unit 16 is further configured to detect if the at least one part of the interventional device lays outside the vasculature presentation shown in the vasculature map and to determine a branching-off location for the interventional device on the vasculature map. The data processing unit 16 is still further configured to adapt the vasculature map in the branching-off location providing an indication of the branching-off location. The output unit 18 is configured to provide an adapted vasculature map.

The term "subject" may also be referred to as individual. The "subject" may further also be referred to as patient, although it is noted that this term does not indicate whether any illness or disease is actually present with the subject.

The vasculature map is also referred to as road map.

As an example, the vasculature map is based on at least one angiographic image of the vasculature structure and comprises the vasculature presentation.

The term "receive and transfer to the data processing unit" can also be referred to as "to provide to the data processing unit" or simply as "to provide".

For example, in the at least one current image of the region of interest, a tip of the interventional device is at least partly visible in the at least one current image.

In an example, the vasculature is a coronary vasculature and the vasculature map is referred to as coronary roadmap.

In an example, the current image refers to a present point in time, i.e. live, e.g. more or less in real-time. The current image can also be referred to as live image.

In an example, the vasculature map and the current image are displayed in an overlaid manner. For example, the vasculature map is overlaid to the current image. In another example, the current image is overlaid to the vasculature map.

In an example, a fine-tuning of the roadmap results in adding secondary vessels to a roadmap after an interventional device like a steering device has navigated into the secondary vessels.

As an example, the location of such disturbing secondary vessel is marked on the roadmap. The clinician can then easily avoid it in later steering. In addition to a marker or indicator, the secondary can also be made visible in the area around the branching-off location and the added details are displayed over the roadmap.

For example, by providing an adapted vasculature map, a clinician is provided with information e.g. in order to avoid steering the device repeatedly into a secondary vessel that is not represented on the initial roadmap.

As an advantage, during navigation, it is detected if the guide wire is driven into a vessel absent from the roadmap. The displayed map is then updated to represent the missing vessel by refining the roadmap extraction locally.

The updated roadmap will help the clinician navigate to pass this region, e.g. by taking additional care not to let the natural dynamics of the guide wire progression drive it into erroneous vessels. The secondary vessel position, e.g. potentially highlighted by a color code, will be kept visible for the navigation of potential additional intervention tools.

Thus, time is saved and the frustration of repeatedly committing the same navigation mistake is omitted or at least limited. Additionally, in an example, roadmaps are corrected where larger vessels would have been omitted during the vasculature map extraction.

The adapted vasculature map also provides advantages when secondary vessels are not displayed on the roadmap.

For example, a roadmap extraction method may be designed to present a more readable roadmap, deprived of too many secondary vessels, and the secondary vessel is thus simply not shown on the roadmap. In such cases where the relevant, large vessels are shown only, and secondary vessels are removed from the roadmap, their presence is made visible by the future steering of the guide wire and the adapted vasculature map.

For example, the secondary vessel of interest is not visible in the angiogram. As an example, the inflow of contrast agent is ruled by different mechanics than the guide wire progression and it may occur that secondary vessels are practically invisible in the angiogram and thus absent from the roadmap. Their presence is made noticeable by the future steering of the guide wire and this information is made visible by the adapted vasculature map.

As an example, wherein the initial roadmap may be incomplete, the adapted vasculature map may show the sub-vessel, in order to warn the clinician that the guide wire, e.g. the guide wire tip may be "attracted" by it

In an example, shown as an option in Fig. 1, a display unit 20 is provided configured to present the adapted vasculature map.

In an example, provided as a further option, for providing the indication of the branching-off location, the data processing unit 16 is configured to fine-tune the vasculature map in a predetermined area around the branching-off location based on the at least one angiographic image. For fine-tuning, the data processing unit 16 is configured to detect more detailed vasculature structures in the at least one angiographic image and to add further details of the vasculature structure to the vasculature map to provide an improved vasculature map.

It is noted that the detection of more detailed vasculature structures in the at least one angiographic image is only provided as one of many possible options.

In another option, for the indication, the data processing unit 16 is configured to fine-tune the vasculature map in a predetermined area around the branching-ofF location based on the at least one angiographic image. For fine-tuning, the data processing unit 16 is configured to retrieve more detailed vasculature structure image data from a further image data source.

In a further example, also provided as an option, the data processing unit 16 is configured to add further details of the vasculature structure to the vasculature map in a highlighted manner.

In an example, provided as a further option, the data processing 16 unit is configured to detect a relevant vascular point or area for the branching-off in the vasculature map. The data processing unit 16 is also configured to transfer the point or area as a relevance location of the branching-off to at least one angiographic image that forms a basis for the vasculature map, and to extract further details from the at least one angiographic image in the relevance location.

In an example, a plurality of angiographic images is provided; and, for each current image, a vasculature map from a matching heart phase is selected.

In an example, the current images are acquired from the same angulation.

In a further example, the angiographic images are shifted to the proper spatial position over the live current image.

In an example, as the vasculature map, a binary vasculature map is extracted, in which the vessels are shown with their outlines.

In an example, the binary calculation is provided for each of the plurality of angiographic images.

In an example, the binary roadmap computation may entail a cleaning step that removes thin secondary vessels from the maps.

In an example, provided as a further option, the data storage unit 14 is configured to provide a plurality of angiographic images. The data processing unit 16 is configured to select a vasculature map from a matching heart phase for each current image.

In an example, provided as a further option, the data processing unit 16 is configured to extract a binary vasculature map as the vasculature map, in which binary vasculature map the vessels are provided with their outlines.

Based on the determined outlines of the vasculature map, a branching-off location can be identified.

In an example, provided as a further option, for the detection of the location of a part of the interventional device, e.g. the tip of the interventional device, in the vasculature map, the data processing unit 16 is configured to segment the current images to identify the interventional device. For the detection if a part of the interventional device, e.g. the tip of the interventional device, lays outside the vasculature shown in the vasculature map, the data processing unit 16 is configured to compare a segmented footprint of the interventional device with the vasculature map.

The segmentation of the interventional device thus provides the basis for the further detection steps in view of the location determination.

In an example, provided as a further option, the vasculature map shows a subject's coronary vessels and the adapted vasculature map with the current image provides an adapted coronary roadmapping.

In an example, provided as a further option, the data processing unit 16 is configured to detect a footprint of the interventional device in the at least one current image, and to transfer the footprint of the interventional device to the vasculature map for the indication of the branching-off location.

For example, the footprint is added in case a fine-tuning based on vascular image data is not possible or is at least not successful.

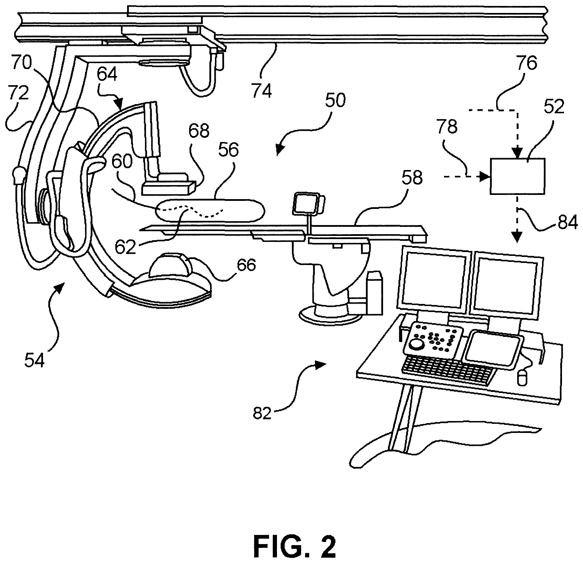

Fig. 2 schematically shows an example of a system 50 for guidance of an interventional device. The system 50 comprises an apparatus 52 for guidance of an interventional device according to one of the preceding examples. The system 50 further comprises a medical imaging device 54. The medical imaging device 54 provides current image data of a region of interest of a vasculature structure of a subject 56.

The subject 56 may be arranged on a subject support 58 of an examination room in a hospital, like a patient table. A catheter 60 or guidewire may be inserted in the vasculature structure of the subject 56, as indicated with a hashed line 62.

In an example, provided as an option for the system 50, the medical imaging device 54 is an X-ray imaging device 64, as shown in Fig. 2, with an X-ray source 66 and an X-ray detector 68, which may be mounted to a movable C-arm structure 70 supported by a movable support 72 suspended from a ceiling mounted rail structure 74.

The X-ray imaging device 64 is configured to provide X-ray images as the at least one current image of the region of interest, which image providing is indicated by a first hashed arrow 76.

The X-ray imaging device 64 may also be configured to provide contrast injected X-ray images as the angiographic images of the vasculature structure for the vasculature map, as indicated by a second hashed arrow 78. The X-ray imaging device 64 thus provides the X-ray image data.

In an example, not further shown, the system comprises a contrast injector for acquiring the contrast injected X-ray images. It is noted that the provision of the contrast injected X-ray images by the X-ray imaging device 64 is provided as an option.

In an example, the X-ray imaging device 64 only provides the X-ray images as the at least one current image of the region of interest and a further imaging source (not shown in detail) is arranged to provide the X-ray images as the angiographic images of the vasculature structure for the vasculature map.

In another example, the X-ray imaging device 64 provides the X-ray images as the at least one current image of the region of interest and also the X-ray images as the angiographic images of the vasculature structure for the vasculature map.

In an example, the X-ray images are live images. For example, the X-ray images are fluoroscopy images of the region of interest.

However, it is noted that also other imaging technologies can be provided for the medical imaging device 54. This relates to providing the at least one current image of the region of interest, and in addition, or alternatively, to providing images for generating the vasculature structure for the vasculature map.

In an example, provided as an option, an interventional device, for example the catheter 60 or guidewire is provided adapted to be moved in a steerable manner within a subject's vasculature structure; and the interventional device is a steering device for interventional procedures.

In an example, the vasculature map shows a subject's coronary vessels and the adapted vasculature map with the current image provides an adapted coronary roadmapping.

Further, as an option, a display 80 is provided next to the subject support 58 on which display 80 the adapted vasculature map can be shown.

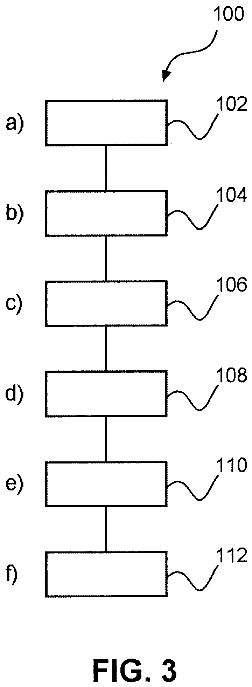

Fig. 3 further shows a control arrangement 82 with additional input devices like a keyboard or a touch pad, together with further monitors. A third hashed arrow 84 indicates a data link between the apparatus 52 for guidance of an interventional device and the control arrangement 82, the data link being provided as an option.

Fig. 3 schematically shows steps of an example of a method 100 for guidance of an interventional device in a vasculature structure. In an example, the method 100 is a computer-implemented method. The method 100 comprises the following steps:
- In a first step 102, also referred to as step a), a vasculature map of a region of interest with a vasculature presentation of a subject's vasculature structure is provided.
- In a second step 104, also referred to as step b), at least one current 2D image of the region of interest is received and transferred to a data processing unit. An interventional device is at least partly visible in the at least one current image when inserted within the vasculature structure in the region of interest
- In a third step 106, also referred to as step c), the vasculature map and the at least one current image are combined.
- In a fourth step 108, also referred to as step d), a location of at least one part of the interventional device is detected in the vasculature map.
- In a fifth step 110, also referred to as step e), it is detected if the at least one part of the interventional device lays outside the vasculature presentation shown in the vasculature map and a branching-off location for the interventional device is determined on the vasculature map.
- In a sixth step 112, also referred to as step f), the vasculature map is adapted in the branching-off location providing an indication of the branching-off location and an adapted vasculature map is presented.

In an example, in step a), a 2D projection of the vasculature map is provided.

In an example, for step d), the current image is segmented.

In an example, in step b), at least one current image is provided. For example, several images are provided, e.g. a sequence of live images is provided.

In an example, in step f), the vasculature map is adapted in an area around the branching-off location.

In an example, for the indication, a point is marked identifying the branching-off location.

In an example, a plurality of angiographic images is provided.

The indication of the branching-off location is also referred to as marking point.

In an example, the method is applied to cardiac roadmapping.

In another example, the method is applied to non-cardiac roadmapping.

In an example, in step f), for the indication, the vasculature map is fine-tuned in a predetermined area around the branching-off location based on the at least one angiographic image.

In an example, for fine-tuning, more detailed vasculature structures are detected in the at least one angiographic image and further details of the vasculature structure are added to the vasculature map resulting in an improved vasculature map.

The adapted vasculature map can thus also be referred to as fine-tuned or improved vasculature map.

The details of the vasculature structure comprise, for example, additional branches of the vasculature structure.

In an example, step e) comprises detecting a relevant vascular branching-off in the vasculature map, and transferring a relevance location to the plurality of angiographic images, and extracting further details in the relevance location in the plurality of angiographic images.

In an example, for step d), the current images are segmented to identify the interventional device; and, for step e), a segmented footprint of the interventional device is compared with the vasculature map.

In an example, the further details of the vasculature structure are added to the vasculature map in a highlighted manner.

In an example, the further details are provided in a different color or pattern.

For example, the added secondary vessel could be shown in a different color, at least when a guide wire is approaching, in order to advert the clinician that his steering device may be attracted by the secondary vessel.

In an example, a signature of the interventional device leaving the vasculature is presented as the warning marker.

In an example, in step f), for the indication, a warning marker is provided identifying the branching-off location.

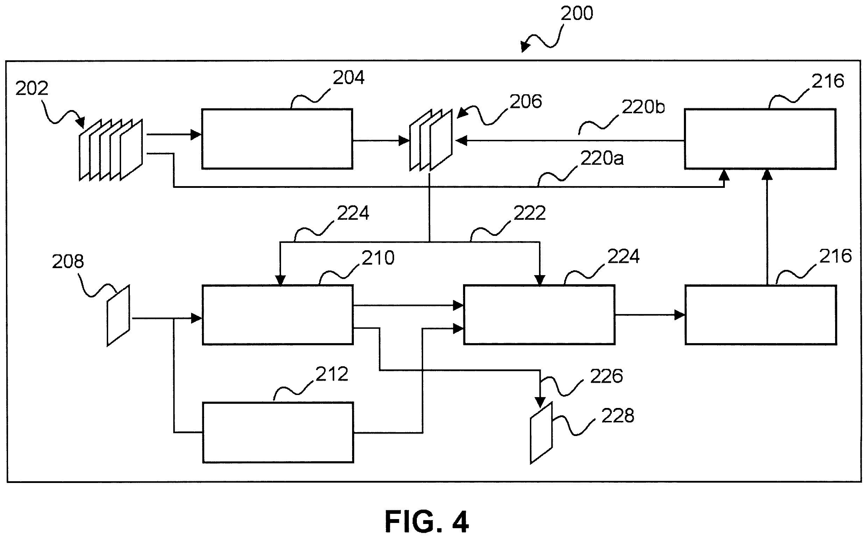

Fig. 4 shows a further example of a method 200 for guidance of an interventional device in a vasculature structure. The method is shown to be cardiac-related, but it is noted that the method is also suitable for other vessel areas. As indicated, a starting point is the general structure of the coronary roadmapping. An angiographic sequence 202 is processed in such a way that i) the best injected cycle it contains is identified, and ii) a binary vasculature map is extracted for each of the identified frames. This step is indicated with a first frame 204. As a result, vascular maps 206 are provided. The binary roadmap computation may entail a cleaning step that removes the thin secondary vessels from the maps.

Then, for each fluoroscopic image 208 (for example, acquired from the same angulation), the vasculature map from the proper heart phase is selected from the vascular maps 206, and shifted to the proper spatial position over the live fluoroscopic image resulting in coronary roadmapping 210.

Further, the steering devices that are potentially present on the fluoroscopic images are detected and segmented, indicated with a further frame 212. This results in knowledge where exactly the device(s) is (are).

Still further, a map branching-off detection 214 is provided. This determines whether steering devices lay outside of the vasculature map, by comparing a segmented footprint of the device on the one hand, with the (shifted) binary vascular maps on the other hand.

In an example, the segmentation is achieved by machine learning techniques using e.g. convolutional networks to learn from annotated examples how to extract them. In another example, traditional computer science techniques are used. As an example, the images are pre-filtered to highlight the elongated structures, then subject to a threshold and discussed on the temporal coherence of the extracted structures.

Furthermore, relevant vascular branching-off determination 216 is provided. For example, if the spatio-temporal matching performed by the coronary roadmapping technique has a sufficient result, the map branching-off detection step is sufficient to identify missing relevant secondary vessels. However, in imperfect matches, a steering wire temporarily outside of the roadmap may yield.

In an example, the spatio-temporal matching performed by the coronary roadmapping technique is perfect, the detecting if the tip of the interventional device lays outside the vasculature is sufficient to identify missing relevant secondary vessels.

In a further example when coronary roadmapping results in imperfect matches, the steering wire is yielded temporarily outside of the roadmap.

In such cases, for example, a number of heuristics are provided to separate the matching errors from the absence of a relevant vessel in the roadmap: For example, in temporal consistency, a matching error will tend to result in "jumps", yielding very different results as soon as the guide wire moves. Further, missing secondary vessels will result in a more stable mismatch, the culprit regions being always the same. For example, in guide wire orientation, for the case of a matching error, the guide wire won't be positioned in the proper vessel, however it will present a similar orientation to it, because both the guide wire and the corresponding vascular map region depict the real vessel orientation. Further, a guide wire engaged in a non-depicted vessel will show a stable orientation, bound to be different from the neighboring large vessel. In an example, a step is provided combining both these heuristics to detect which of the map branching-offs correspond to a vessel absent from the roadmap. It will also indicate on the vessel map where a sub-vessel is missing.

If a missing vessel has been detected and its localization has been determined, the vessel maps used in the roadmapping are refined 218. As an example, the selected angiograms are reprocessed, as indicated with first and second arrows 220a and 220b, with a closest attention to the secondary vessels that may be present in the area. For instance, a smallest threshold can be used to locally extract the vessel. Or the thin vessel cleaning step can be disabled in the neighborhood.

At the end of this step, it is checked 222, also in frame 214, whether the local modification satisfactorily accounts for the map's branching-off(s) being observed. If not, for instance because the sub-vessel of interest was not visible on the original angiogram, the roadmap is automatically re-edited 224. Then the footprint of the guide wire that was following a branch of the vasculature structure is added, preferably in a different color. This will complete a roadmap 226 which is then provided 228, accounting for the observed wire branching-off. This will serve the need of helping to avoid repeated leaving the roadmap and e.g. unintentionally following branches of the vasculature structure at that point, even if the added information does not perfectly depict the real secondary vessel.

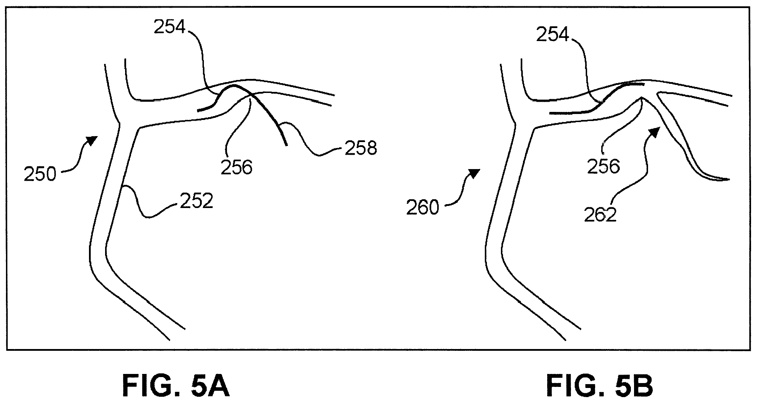

Fig. 5a shows a vasculature map 250 of a region of interest of a subject's vasculature structure. The vasculature map 250 is provided as a roadmap in which outlines of primary vessels 252 are shown. Further, e.g. by overlaying a current image, a distal end portion of an interventional device like a guidewire 254 is shown inserted in the vasculature structure. The guidewire 254 is shown as leaving the roadmap, or branching-off from the shown vasculature structure at a branching-off location 256, in which a part 258 seems to be located outside the vessel structure. Hence, the roadmap may be considered as an incomplete roadmap.

Fig. 5b shows an adapted vasculature map 260 as an updated roadmap in which the branching-off location is indicated, for example by locally refining the roadmap. As shown in Fig. 5b, the vascular structure 252 is shown with outlines and the device 254 is present in the vascular structure. However, the vascular structure 252 is provided with an added detail 262 of a secondary vessel branching off the main vessel at the branching-off location 256 determined based on the image shown in Fig. 5a. The added detail can thus act as a visible warning. A user operating the guidewire 254 can thus steer past the branching-off location 256 in a facilitated manner. The adapted vasculature map 260 can also be referred to as completed roadmap.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

**1.** An apparatus (10) for guidance of an interventional device, the apparatus comprising:
- an input unit (12);
- a data storage unit (14);
- a data processing unit (16); and
- an output unit (18);
wherein the data storage unit is configured to provide a vasculature map of a region of interest with a vasculature presentation of a subject's vasculature structure;
wherein the input unit is configured to receive and transfer to the data processing unit at least one current image of the region of interest, wherein at least one part of an interventional device is at least partly visible in the at least one current image when inserted within the vasculature structure in the region of interest;
wherein the data processing unit is configured to combine the vasculature map and the at least one current image; to detect a location of the at least one part of the interventional device in the vasculature map; to detect if the at least one part of the interventional device lays outside the vasculature presentation shown in the vasculature map and to determine a branching-off location for the interventional device on the vasculature map; to adapt the vasculature map in the branching-off location providing an indication of the branching-off location; and
wherein the output unit is configured to provide an adapted vasculature map.

**2.** Device according to claim 1, wherein, for providing the indication of the branching-off location, the data processing unit is configured to fine-tune the vasculature map in a predetermined area around the branching-off location based on the at least one angiographic image; and
wherein, for fine-tuning, the data processing unit is configured to detect more detailed vasculature structures in the at least one angiographic image and to add further details of the vasculature structure to the vasculature map to provide an improved vasculature map.

**3.** Device according to one of the preceding claims, wherein the data processing unit is configured to detect a relevant vascular point or area for the branching-off in the vasculature map; to transfer the point or area as a relevance location of the branching-off to at least one angiographic image that forms a basis for the vasculature map; and to extract further details from the at least one angiographic image in the relevance location.

**4.** Device according to one of the preceding claims, wherein the data storage unit is configured to provide a plurality of angiographic images; and
wherein the data processing unit is configured to select a vasculature map from a matching heart phase for each current image.

**5.** Device according to one of the preceding claims, wherein the data processing unit is configured to extract a binary vasculature map as the vasculature map, in which binary vasculature map the vessels are provided with their outlines.

**6.** Device according to one of the preceding claims, wherein the data processing unit is configured, for the detection of the location of the interventional device in the vasculature map, to segment the current images to identify the at least a part of the interventional device; and, for the detection if a part of the interventional device lays outside the vasculature shown in the vasculature map, to compare a segmented footprint of the interventional device with the vasculature map.

**7.** Device according to one of the preceding claims, wherein the vasculature map shows a subject's coronary vessels and the adapted vasculature map with the current image provides an adapted coronary roadmapping.

**8.** Device according to one of the preceding claims, wherein the data processing unit is configured to detect a footprint of the interventional device in the at least one current image; and to transfer the footprint of the interventional device to the vasculature map for the indication of the branching-off location.

**9.** Device according to claim 1, wherein a display unit (20) is provided configured to present the adapted vasculature map.

**10.** A system (50) for guidance of an interventional device, the system comprising:
- an apparatus (52) for guidance of an interventional device according to one of the preceding claims; and
- a medical imaging device (54);
wherein the medical imaging device provides current image data of a region of interest of a subject's vasculature structure.

**11.** System according to claim 10, wherein the medical imaging device is an X-ray imaging device;
wherein the X-ray imaging device is configured to provide X-ray images as the at least one current image of the region of interest; and
wherein the X-ray imaging device is configured to provide contrast injected X-ray images as the angiographic images of the vasculature structure for the vasculature map.
System according to claim 10 or 11, wherein an interventional device is provided adapted to be moved in a steerable manner within a subject's vasculature structure; and
wherein the interventional device is a steering device for interventional procedures.

**12.** A computer-implemented method (100) for guidance of an interventional device in a vasculature structure, the method comprising the following steps:
a) providing (102) a vasculature map of a region of interest with a vasculature presentation of a subject's vasculature structure;
b) receiving and transferring to a data processing unit (104) at least one current 2D image of the region of interest, wherein at least one part of an interventional device is at least partly visible in the at least one current image when inserted within the vasculature structure in the region of interest;
c) combining (106) the vasculature map and the at least one current image;
d) detecting (108) a location of the at least one part of the interventional device in the vasculature map;
e) detecting (110) if the at least one part of the interventional device lays outside the vasculature presentation shown in the vasculature map and determining a branching-off location for the interventional device on the vasculature map; and
f) adapting (112) the vasculature map in the branching-off location providing an indication of the branching-off location and presenting an adapted vasculature map.

**14.** A computer program element for controlling an apparatus according to one of the claims 1 to 9 or a system according to one of the claims 10 to 12, which apparatus or system, when being executed by a processing unit, is adapted to perform the method steps of claim 13.

**15.** A computer readable medium having stored the program element of claim 14.
